# EUROPEAN PATENT APPLICATION

(11) **EP 4 786 474 A1**
(43) Date of publication of application: **05.08.2026**
(21) Application number: 24870936.2
(22) Date of filing: 27.09.2024
(51) Int. Cl.: C07D 519/00, C07D 403/00, C07D 487/00, A61K 31/395, A61K 31/517, A61P 35/00

(54) **CRYSTALLINE FORM OF QUINAZOLINE DERIVATIVE AND PREPARATION METHOD THEREFOR**

(30) Priority: 27.09.2023 CN 202311254943
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: YANG, Shibo, Shanghai 200245 (CN); WANG, Jie, Lianyungang, Jiangsu 222047 (CN); SHAO, Qiyun, Shanghai 200245 (CN); DU, Zhenxing, Lianyungang, Jiangsu 222047 (CN)
(74) Representative: dompatent
(86) International application number: PCT/CN2024/121644
(87) International publication number: WO 2025/067398

(57) **Abstract**

A crystalline form of a quinazoline derivative and a preparation method therefor. Specifically, provided is an E crystal form, D crystal form, and H crystal form of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, which have good stability and can be better used for clinical treatment.

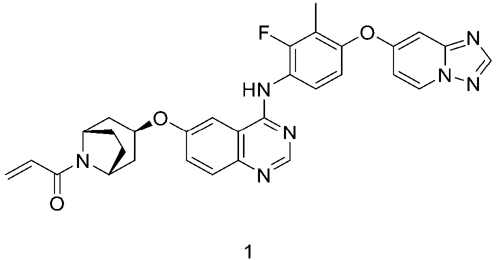

## Description

### The present application claims priority to:

Application No. CN202311254943.2, filed on September 27, 2023.

### TECHNICAL FIELD

The present disclosure pertains to the technical field of pharmaceutics, and relates to a crystal form of a quinazoline derivative and a method for preparing same.

### BACKGROUND

Human epidermal growth factor receptor 2 (HER2; Neu, ERBB2) is a member of the type I receptor tyrosine kinase family, which also includes EGFR (ERBB1), HER3 (ERBB3), and HER4 (ERBB4). So far, no ligand that can directly bind to HER2 has been found in the human body. HER2 must form homodimers or heterodimers with other members of the family (e.g., HER3). After dimerization, HER2 undergoes conformational changes, activating the intracellular tyrosine kinase activity, which in turn activates downstream pathways (MAPK signaling pathway and PI3K/AKT signaling pathway), thereby exerting corresponding physiological effects.

According to statistics, about 2%-4% of patients with lung cancer carry activating mutations in HER2 exon 20. Currently, clinically approved ERBB-targeting tyrosine kinase inhibitors are almost ineffective in these patients, primarily due to EGFR wild-type-mediated dose-limiting toxicity. Allitinib, ibrutinib, neratinib, poziotinib, and pyrotinib are known broad-spectrum ERBB inhibitors of mutant HER2 exon 20. However, in clinical practice, due to limitations in effective dose, afatinib and other broad-spectrum ERBB inhibitors have shown only limited efficacy in NSCLC patients with HER2 exon 20 mutations.

PCT/CN2023/084265 provides a novel inhibitor targeting HER2 exon 20 mutations that is selective for EGFR wild type. It is chemically named 1-(*endo*-3-((4-((4-([1,2,4]triazolo[1,5-*a*]pyridin-7-yloxy)-2-fluoro-3-methylphenyl)amino)quinazolin-6-yl)oxy)-8-azabicyclo[3.2.1]octan-8-yl)prop-2-en-1-one, and has a structure represented by formula 1,

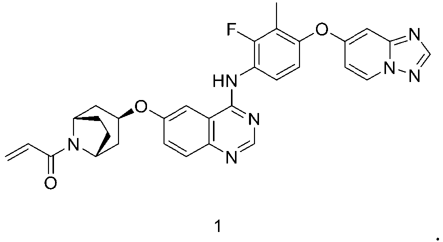

A crystal form used as an active pharmaceutical ingredient often influences the chemical stability of the drug. Variations in crystallization conditions and storage conditions may lead to changes in the crystal form structure of the compound, sometimes accompanied by the generation of other crystal forms. Generally, amorphous drug products lack a regular crystal form structure and often have other defects, such as poor product stability, fine particles, difficulty in filtration, tendency to agglomerate, and poor flowability. Polymorphs of a drug impose different requirements on product storage, production, and scale-up. Therefore, it is necessary to extensively study the crystal forms of the aforementioned compound and to improve various properties of the aforementioned compound.

### SUMMARY

The present disclosure provides a new crystal form of the compound represented by formula 1, which has good stability and better clinical prospects.

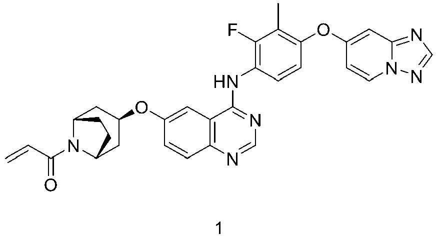

The present disclosure provides a crystal form A of the compound represented by formula 1, wherein an X-ray powder diffraction pattern expressed in terms of 2*θ* angles, which are diffraction angles, of the crystal form has characteristic peaks at 8.905, 9.836, 12.375, 13.207, 17.814, 18.265, 19.863, and 26.491.

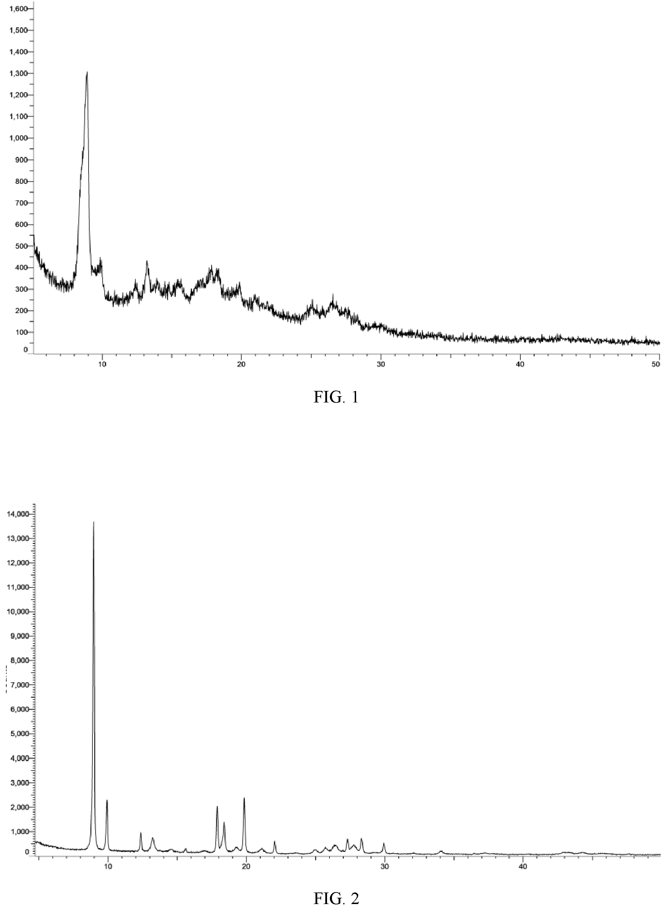

In some embodiments, the X-ray powder diffraction pattern expressed in terms of 2*θ* angles, which are diffraction angles, of the crystal form of the compound represented by formula 1 is shown in FIG. 1.

The present disclosure further provides a method for preparing the crystal form A of the compound represented by formula 1, comprising dissolving the compound represented by formula 1 in methanol/ethyl acetate = 1:10 (v/v) and evaporating the solvent for crystallization.

The present disclosure further provides a crystal form B of the compound represented by formula 1, wherein an X-ray powder diffraction pattern expressed in terms of 2*θ* angles, which are diffraction angles, of the crystal form has characteristic peaks at 8.963, 9.926, 17.902, 18.404, 19.850, and 22.049.

In some embodiments, the X-ray powder diffraction pattern expressed in terms of 2*θ* angles, which are diffraction angles, of the crystal form B of the compound represented by formula 1 has characteristic peaks at 8.963, 9.926, 12.368, 13.219, 17.902, 18.404, 19.850, and 22.049.

In some embodiments, the X-ray powder diffraction pattern expressed in terms of 2*θ* angles, which are diffraction angles, of the crystal form B of the compound represented by formula 1 is shown in FIG. 2.

The present disclosure further provides a method for preparing the crystal form B of the compound represented by formula 1, comprising adding the crystal form A of the compound represented by formula 1 to methanol and stirring.

The present disclosure further provides a crystal form C of the compound represented by formula 1, wherein an X-ray powder diffraction pattern expressed in terms of 2*θ* angles, which are diffraction angles, of the crystal form has characteristic peaks at 6.071, 8.758, 13.787, 15.348, 17.522, and 21.621.

In some embodiments, the X-ray powder diffraction pattern expressed in terms of 2*θ* angles, which are diffraction angles, of the crystal form C of the compound represented by formula 1 has characteristic peaks at 6.071, 8.758, 9.708, 13.787, 15.348, 17.522, 18.228, and 21.621.

In some embodiments, the X-ray powder diffraction pattern expressed in terms of 2*θ* angles, which are diffraction angles, of the crystal form C of the compound represented by formula 1 has characteristic peaks at 6.071, 8.758, 9.708, 13.787, 15.348, 17.522, 18.228, 18.695, 21.621, 24.874, and 25.724.

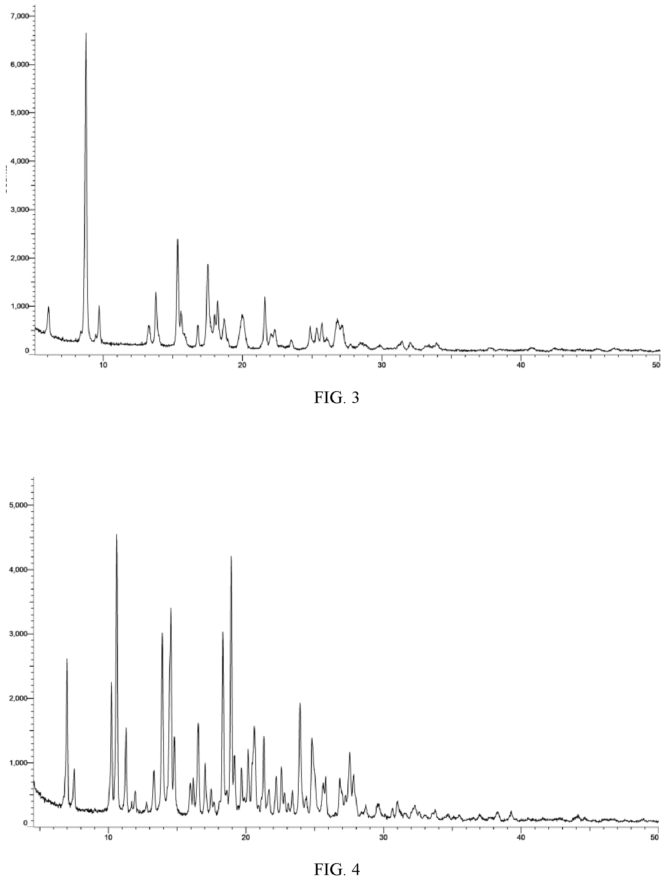

In some embodiments, the X-ray powder diffraction pattern expressed in terms of 2*θ* angles, which are diffraction angles, of the crystal form C of the compound represented by formula 1 is shown in FIG. 3.

The present disclosure further provides a method for preparing the crystal form C of the compound represented by formula 1, comprising:
method I: dissolving the compound represented by formula 1 in a solvent A and stirring, wherein the solvent A is a solvent selected from the group consisting of ethanol, *n*-propanol, ethyl acetate/ethanol = 1:1 (v/v), and tetrahydrofuran/ethanol = 2:1 (v/v);
method II: dissolving the compound represented by formula 1 in *n*-propanol, adding *n*-heptane, and stirring.

The present disclosure further provides a crystal form D of the compound represented by formula 1, wherein an X-ray powder diffraction pattern expressed in terms of 2*θ* angles, which are diffraction angles, of the crystal form has characteristic peaks at 6.989, 10.610, 13.927, 14.544, 18.332, 18.930, and 23.940.

In some embodiments, the X-ray powder diffraction pattern expressed in terms of 2*θ* angles, which are diffraction angles, of the crystal form D of the compound represented by formula 1 has characteristic peaks at 6.989, 7.512, 10.223, 10.610, 11.281, 13.927, 14.544, 14.805, 16.535, 18.332, 18.930, and 23.940.

In some embodiments, the X-ray powder diffraction pattern expressed in terms of 2*θ* angles, which are diffraction angles, of the crystal form D of the compound represented by formula 1 has characteristic peaks at 6.989, 7.512, 10.223, 10.610, 11.281, 13.310, 13.927, 14.544, 14.805, 16.535, 17.044, 18.332, 18.930, 20.617, 21.313, and 23.940.

In some embodiments, the X-ray powder diffraction pattern expressed in terms of 2*θ* angles, which are diffraction angles, of the crystal form D of the compound represented by formula 1 is shown in FIG. 4.

The present disclosure further provides a method for preparing the crystal form D of the compound represented by formula 1, comprising:
method I: adding the compound represented by formula 1 to a solvent V and stirring, wherein the solvent V is one or more solvents selected from the group consisting of water, isopropyl acetate, toluene, and ethyl acetate/*n*-heptane = 1:1 (v/v);
method II: dissolving the compound represented by formula 1 in a solvent VI and stirring, wherein the solvent VI is one or more solvents selected from the group consisting of dichloromethane, tetrahydrofuran, water/isopropanol = 1:9 (v/v), and water/acetone = 1:9 (v/v);
method III: dissolving the compound represented by formula 1 in a solvent VII, adding a solvent VIII, and stirring, wherein when the VII is a solvent selected from the group consisting of *n-*propanol, propylene glycol methyl ether, and tetrahydrofuran, the solvent VIII added is water; when the VII is selected from the group consisting of dichloromethane, the solvent VIII added is a solvent selected from the group consisting of ethyl acetate and methyl *tert*-butyl ether;
method IV: dissolving the compound represented by formula 1 in a solvent IX and volatilizing for crystallization, wherein the IX is one or more solvents selected from the group consisting of propylene glycol methyl ether and *N*,*N*-dimethylformamide.

The present disclosure further provides a crystal form E of the compound represented by formula 1, wherein an X-ray powder diffraction pattern expressed in terms of 2*θ* angles, which are diffraction angles, of the crystal form has characteristic peaks at 10.080, 14.012, 17.465, 18.635, 20.135, and 24.921.

In some embodiments, the X-ray powder diffraction pattern expressed in terms of 2*θ* angles, which are diffraction angles, of the crystal form E of the compound represented by formula 1 has characteristic peaks at 10.080, 11.701, 12.776, 14.012, 15.958, 17.465, 17.696, 20.135, 21.144, 23.088, and 24.921.

In some embodiments, the X-ray powder diffraction pattern expressed in terms of 2*θ* angles, which are diffraction angles, of the crystal form E of the compound represented by formula 1 has characteristic peaks at 10.080, 10.296, 11.701, 12.776, 14.012, 14.286, 15.958, 16.507, 17.177, 17.465, 17.696, 18.635, 19.111, 19.828, 20.135, 21.144, 23.088, 24.921, 26.493, and 31.991.

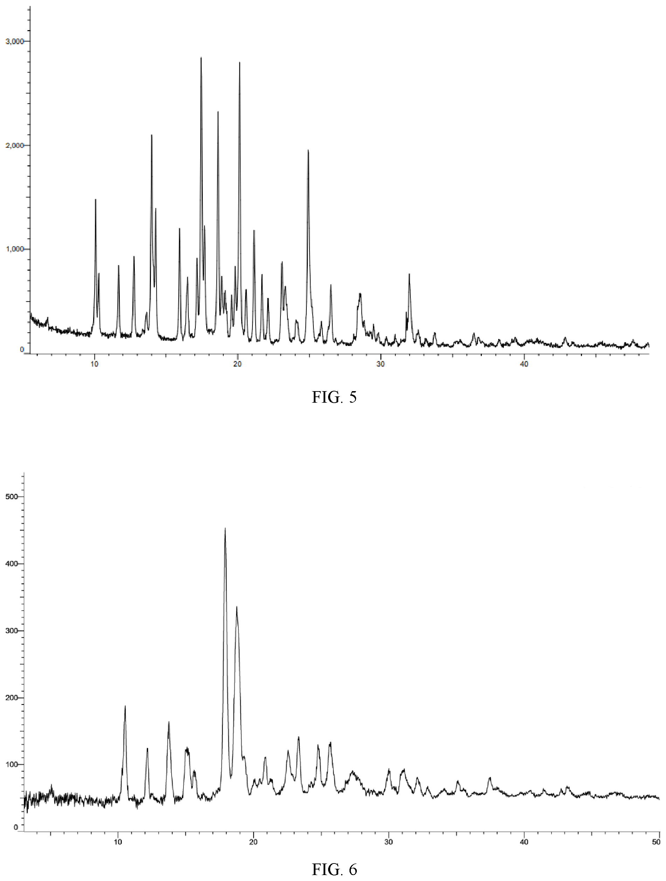

In some embodiments, the X-ray powder diffraction pattern expressed in terms of 2*θ* angles, which are diffraction angles, of the crystal form E of the compound represented by formula 1 is shown in FIG. 5.

The present disclosure further provides a method for preparing the crystal form E of the compound represented by formula 1, comprising:
method I: adding the compound represented by formula 1 to a solvent I and stirring, wherein the solvent I is one or more solvents selected from the group consisting of ethyl acetate, acetonitrile, *n*-heptane, methyl *tert*-butyl ketone, isopropanol, 1,4-dioxane, 2-methyltetrahydrofuran, acetone, methyl *tert*-butyl ether, cyclohexane, *n*-hexane, and isopropyl ether;
method II: dissolving the compound represented by formula 1 in a solvent II, adding a solvent III, and stirring, wherein when the II is selected from the group consisting of *n*-propanol, the solvent III added is a solvent selected from the group consisting of ethyl acetate, acetonitrile, methyl *tert-*butyl ether, and methyl isobutyl ketone; when the II is selected from the group consisting of propylene glycol methyl ether and tetrahydrofuran, the solvent III added is a solvent selected from the group consisting of ethyl acetate, acetonitrile, methyl *tert*-butyl ether, methyl isobutyl ketone, and *n*-heptane; when the II is selected from the group consisting of dichloromethane, the solvent III added is a solvent selected from the group consisting of isopropyl ether, methyl isobutyl ketone, and *n*-heptane.

The present disclosure further provides a crystal form F of the compound represented by formula 1, wherein an X-ray powder diffraction pattern expressed in terms of 2*θ* angles, which are diffraction angles, of the crystal form has characteristic peaks at 10.529, 12.617, 13.759, 17.919, and 18.778.

In some embodiments, the X-ray powder diffraction pattern expressed in terms of 2*θ* angles, which are diffraction angles, of the crystal form F of the compound represented by formula 1 has characteristic peaks at 10.529, 12.617, 13.759, 17.919, 18.778, 22.566, 23.328, 24.788, and 25.666. In some embodiments, the X-ray powder diffraction pattern expressed in terms of 2*θ* angles, which are diffraction angles, of the crystal form F of the compound represented by formula 1 has characteristic peaks at 10.529, 12.617, 13.759, 17.919, 18.778, 20.887, 22.566, 23.328, 24.788, 25.666, 30.028, and 31.080.

In some embodiments, the X-ray powder diffraction pattern expressed in terms of 2*θ* angles, which are diffraction angles, of the crystal form F of the compound represented by formula 1 is shown in FIG. 6.

The present disclosure further provides a method for preparing the crystal form F of the compound represented by formula 1, comprising adding the compound represented by formula 1 to a solvent B and stirring, wherein the solvent B is a solvent selected from the group consisting of methanol, water/methanol = 1:1 (v/v), water/methanol = 1:9 (v/v), and acetonitrile/methanol = 1:1 (v/v). The present disclosure further provides a crystal form G of the compound represented by formula 1, wherein an X-ray powder diffraction pattern expressed in terms of 2*θ* angles, which are diffraction angles, of the crystal form has characteristic peaks at 10.437, 11.093, 13.989, 17.124, 17.620, 21.374, and 22.677.

In some embodiments, the X-ray powder diffraction pattern expressed in terms of 2*θ* angles, which are diffraction angles, of the crystal form G of the compound represented by formula 1 has characteristic peaks at 10.437, 11.093, 13.989, 17.124, 17.620, 19.601, 19.929, 21.374, and 22.677. In some embodiments, the X-ray powder diffraction pattern expressed in terms of 2*θ* angles, which are diffraction angles, of the crystal form G of the compound represented by formula 1 has characteristic peaks at 10.437, 11.093, 11.362, 11.881, 12.161, 13.989, 17.124, 17.620, 19.601, 19.929, 21.374, and 22.677.

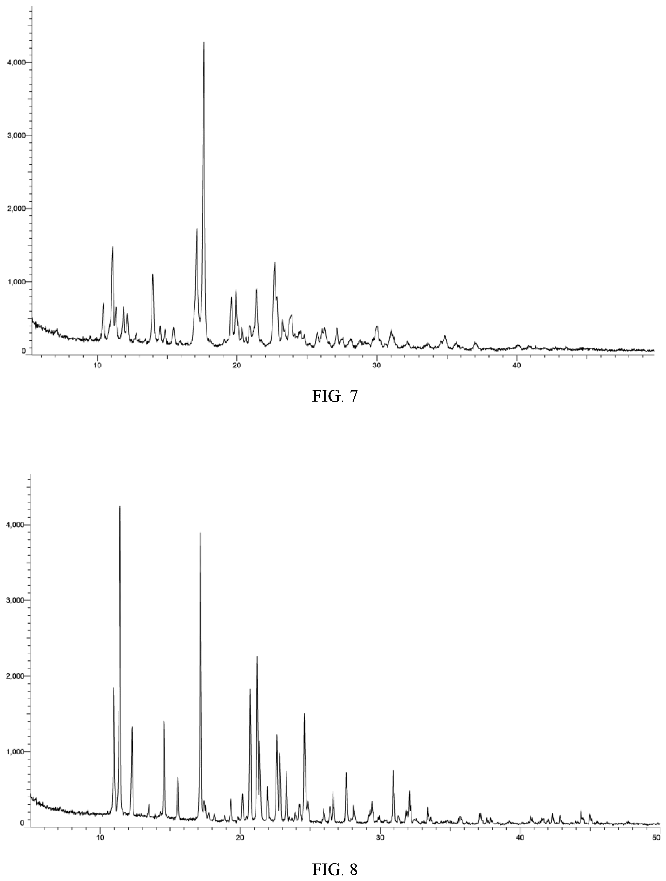

In some embodiments, the X-ray powder diffraction pattern expressed in terms of 2*θ* angles, which are diffraction angles, of the crystal form G of the compound represented by formula 1 is shown in FIG. 7.

The present disclosure further provides a method for preparing the crystal form G of the compound represented by formula 1, comprising: adding the crystal form F of the compound represented by formula 1 to a solvent C and stirring, wherein the solvent C is a solvent selected from the group consisting of tetrahydrofuran, 2-butanone, and methyl isobutyl ketone.

The present disclosure further provides a crystal form H of the compound represented by formula 1, wherein an X-ray powder diffraction pattern expressed in terms of 2*θ* angles, which are diffraction angles, of the crystal form has characteristic peaks at 10.963, 11.405, 17.174, 20.715, 21.265, and 24.600.

In some embodiments, the X-ray powder diffraction pattern expressed in terms of 2*θ* angles, which are diffraction angles, of the crystal form H of the compound represented by formula 1 has characteristic peaks at 10.963, 11.405, 12.273, 14.569, 17.174, 20.715, 21.265, 21.328, 22.626, 22.834, and 24.600.

In some embodiments, the X-ray powder diffraction pattern expressed in terms of 2*θ* angles, which are diffraction angles, of the crystal form H of the compound represented by formula 1 has characteristic peaks at 10.963, 11.405, 12.273, 14.569, 17.174, 20.715, 21.265, 21.328, 22.626, 22.834, 24.600, 27.581, and 30.944.

In some embodiments, the X-ray powder diffraction pattern expressed in terms of 2*θ* angles, which are diffraction angles, of the crystal form H of the compound represented by formula 1 is shown in FIG. 8.

The present disclosure further provides a method for preparing the crystal form H of the compound represented by formula 1, comprising:
method I: dissolving the compound represented by formula 1 in water/ethanol = 0.7:9.3 (v/v), isopropanol, or 2-butanone, and stirring;
method II: dissolving the compound represented by formula 1 in dichloromethane, adding acetonitrile, and stirring.

The present disclosure further provides a crystal form I of the compound represented by formula 1, wherein an X-ray powder diffraction pattern expressed in terms of 2*θ* angles, which are diffraction angles, of the crystal form has characteristic peaks at 8.451, 9.371, 12.950, 15.468, 24.693, and 26.353.

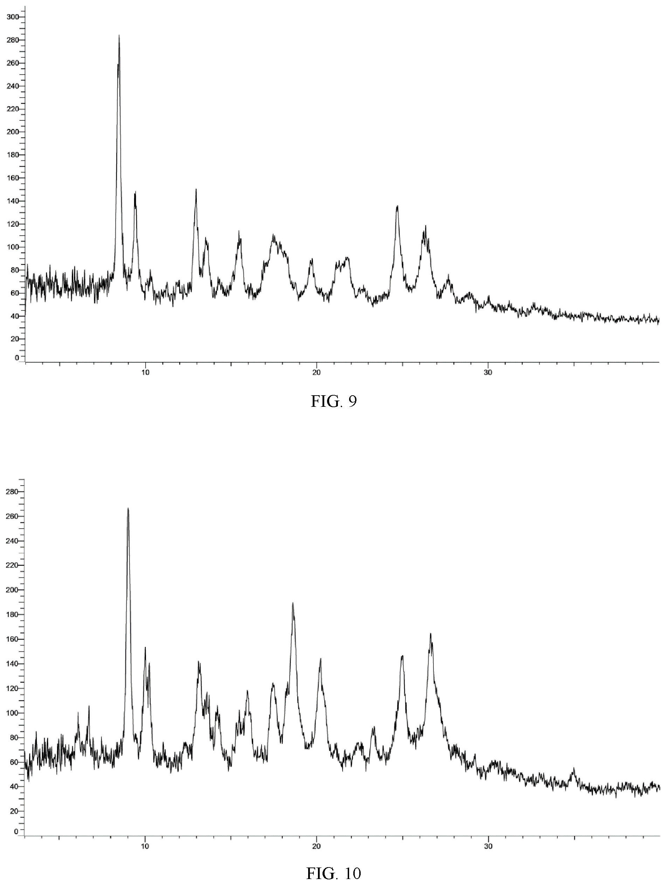

In some embodiments, the X-ray powder diffraction pattern expressed in terms of 2*θ* angles, which are diffraction angles, of the crystal form I of the compound represented by formula 1 has characteristic peaks at 8.451, 9.371, 12.950, 13.556, 15.468, 17.911, 19.681, 24.693, and 26.353. In some embodiments, the X-ray powder diffraction pattern expressed in terms of 2*θ* angles, which are diffraction angles, of the crystal form I of the compound represented by formula 1 is shown in FIG. 9.

The present disclosure further provides a method for preparing the crystal form I of the compound represented by formula 1, comprising adding the compound represented by formula 1 to water/methanol = 8:2 (v/v) and stirring.

The present disclosure further provides a crystal form J of the compound represented by formula 1, wherein an X-ray powder diffraction pattern expressed in terms of 2*θ* angles, which are diffraction angles, of the crystal form has characteristic peaks at 9.020, 10.008, 17.472, 18.631, 20.232, 24.977, and 26.623.

In some embodiments, the X-ray powder diffraction pattern expressed in terms of 2*θ* angles, which are diffraction angles, of the crystal form J of the compound represented by formula 1 has characteristic peaks at 9.020, 10.008, 13.149, 15.973, 17.472, 18.631, 20.232, 23.333, 24.977, and 26.623.

In some embodiments, the X-ray powder diffraction pattern expressed in terms of 2*θ* angles, which are diffraction angles, of the crystal form J of the compound represented by formula 1 is shown in FIG. 10.

The present disclosure further provides a method for preparing the crystal form J of the compound represented by formula 1, comprising adding the compound represented by formula 1 to water/methanol = 8:2 (v/v) and stirring.

The present disclosure further provides a crystal form K of the compound represented by formula 1, wherein an X-ray powder diffraction pattern expressed in terms of 2*θ* angles, which are diffraction angles, of the crystal form has characteristic peaks at 5.814, 11.642, 13.010, 15.159, 20.326, and 23.543.

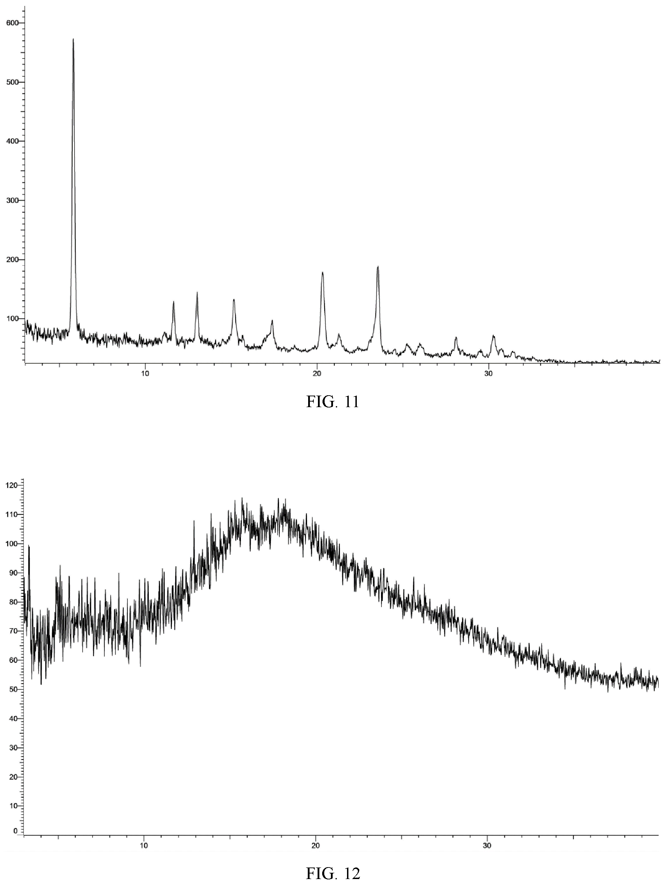

In some embodiments, the X-ray powder diffraction pattern expressed in terms of 2*θ* angles, which are diffraction angles, of the crystal form K of the compound represented by formula 1 is shown in FIG. 11.

The present disclosure further provides a method for preparing the crystal form K of the compound represented by formula 1, comprising dissolving the compound represented by formula 1 in acetonitrile and volatilizing for crystallization.

The present disclosure further provides a crystal form L of the compound represented by formula 1, wherein an X-ray powder diffraction pattern expressed in terms of 2*θ* angles, which are diffraction angles, of the crystal form has characteristic peaks at 7.471, 10.514, 13.476, 16.708, 18.749, and 21.243.

In some embodiments, the X-ray powder diffraction pattern expressed in terms of 2*θ* angles, which are diffraction angles, of the crystal form L of the compound represented by formula 1 has characteristic peaks at 5.350, 7.471, 10.514, 13.476, 13.876, 16.708, 18.749, 21.243, 23.202, and 26.097.

In some embodiments, the X-ray powder diffraction pattern expressed in terms of 2*θ* angles, which are diffraction angles, of the crystal form L of the compound represented by formula 1 has characteristic peaks at 5.350, 7.471, 10.514, 12.171, 13.476, 13.876, 15.802, 16.708, 17.950, 18.749, 21.243, 21.932, 23.202, 24.826, and 26.097.

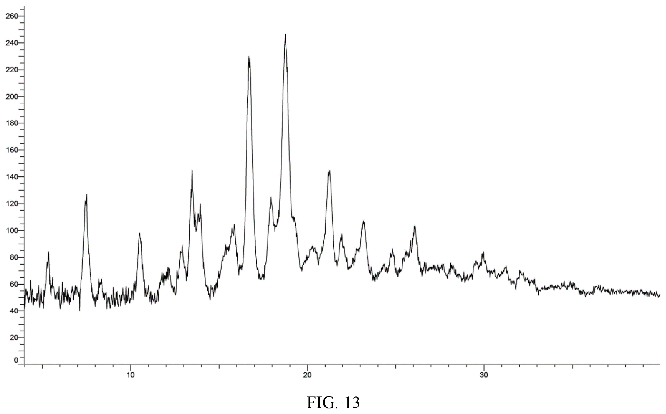

In some embodiments, the X-ray powder diffraction pattern expressed in terms of 2*θ* angles, which are diffraction angles, of the crystal form L of the compound represented by formula 1 is shown in FIG. 13.

The present disclosure further provides a method for preparing the crystal form L of the compound represented by formula 1, comprising steps of dissolving the compound represented by formula 1 in acetone and stirring.

The present disclosure further provides a pharmaceutical composition, comprising the aforementioned crystal form A, crystal form B, crystal form C, crystal form D, crystal form E, crystal form F, crystal form G, crystal form H, crystal form I, crystal form J, crystal form K, or crystal form L, and optionally a pharmaceutically acceptable auxiliary material selected from the group consisting of pharmaceutically acceptable excipients.

The present disclosure further provides a pharmaceutical composition, which is prepared from the aforementioned crystal form A, crystal form B, crystal form C, crystal form D, crystal form E, crystal form F, crystal form G, crystal form H, crystal form I, crystal form J, crystal form K, or crystal form L, and optionally a pharmaceutically acceptable excipient.

The present disclosure further provides a method for preparing a pharmaceutical composition, comprising: a step of mixing the aforementioned crystal form A, crystal form B, crystal form C, crystal form D, crystal form E, crystal form F, crystal form G, crystal form H, crystal form I, crystal form J, crystal form K, or crystal form L with a pharmaceutically acceptable excipient.

The present disclosure further provides use of the aforementioned crystal form A, crystal form B, crystal form C, crystal form D, crystal form E, crystal form F, crystal form G, crystal form H, crystal form I, crystal form J, crystal form K, or crystal form L, or the aforementioned composition in the manufacture of a medicament for preventing and/or treating a cancer.

In the use of the present disclosure, the cancer is selected from the group consisting of breast cancer, ovarian cancer, gastric cancer, colorectal cancer, cervical cancer, endometrial cancer, bladder cancer, brain cancer, epithelial cancer, esophageal cancer, mesothelioma, nasopharyngeal carcinoma, oral cancer, thyroid cancer, skin cancer, squamous cell carcinoma, synovioma, and sweat gland carcinoma; preferably, the cancer is selected from the group consisting of breast cancer, ovarian cancer, cervical cancer, endometrial cancer, gastric cancer, colorectal cancer, and lung cancer.

As used herein, "2*θ* or 2*θ* angle" refers to a diffraction angle; *θ* refers to the Bragg angle in ° or degrees; the 2*θ* of each characteristic peak has a margin of error of ±0.20 (including the case that a number having more than 1 decimal place is rounded), specifically -0.20, -0.19, -0.18, -0.17, - 0.16, -0.15, -0.14, -0.13, -0.12, -0.11, -0.10, -0.09, -0.08, -0.07, -0.06, -0.05, -0.04, -0.03, -0.02, - 0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

In the present disclosure, numerical values such as the content of related substances are data obtained by measurement and calculation, and there is inevitably a certain degree of error. In general, ±10% is a reasonable margin of error. There is a certain degree of error change depending on the context where it is used, and the error change is no more than ±10% and may be ±9%, ±8%, ±7%, ±6%, ±5%, ±4%, ±3%, ±2%, or ±1%, preferably ±5%.

The starting material used in the methods for preparing the crystal forms of the present disclosure may be any form of the compound, and the specific forms include, but are not limited to: an amorphous form, any crystal form, a hydrate, a solvate, and the like.

In the present disclosure, the drying temperature is generally 25 °C-100 °C, preferably 40 °C-70 °C, and the drying may be performed under atmospheric pressure or reduced pressure.

Methods for the crystallization described in the present disclosure include room-temperature crystallization, cooling crystallization, crystallization by volatilizing the solvent, crystallization induced by adding a seed crystal, and the like, wherein the cooling temperature is selected from the group consisting of 65 °C or lower, preferably from the group consisting of -10 °C to 60 °C, and the crystallization process may involve stirring.

As used herein, "differential scanning calorimetry" or "DSC" refers to measuring the temperature difference and heat flow difference between a sample and a reference substance during a ramping or thermostatic process, in order to characterize all the physical changes and chemical changes related to the thermal effect, thereby obtaining phase change information about the sample.

According to the description of hygroscopicity characteristics and the definition of hygroscopic weight gain in "General Rule 9103, Guidelines for Hygroscopicity Trials for Pharmaceuticals" in the Chinese Pharmacopoeia, Volume IV, 2015 Edition:
deliquescent: sufficient water is absorbed to form a liquid;
extremely hygroscopic: the hygroscopic weight gain is not less than 15%;
hygroscopic: the hygroscopic weight gain is less than 15% but not less than 2%;
slightly hygroscopic: the hygroscopic weight gain is less than 2% but not less than 0.2%;
nonhygroscopic or practically nonhygroscopic: the hygroscopic weight gain is less than 0.2%.

As used herein, "excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or domestic animals.

### BRIEF DESCRIPTION OF THE DRAWINGS

FIG. 1 shows an XRPD pattern of the crystal form A of compound 1.
FIG. 2 shows an XRPD pattern of the crystal form B of compound 1.
FIG. 3 shows an XRPD pattern of the crystal form C of compound 1.
FIG. 4 shows an XRPD pattern of the crystal form D of compound 1.
FIG. 5 shows an XRPD pattern of the crystal form E of compound 1.
FIG. 6 shows an XRPD pattern of the crystal form F of compound 1.
FIG. 7 shows an XRPD pattern of the crystal form G of compound 1.
FIG. 8 shows an XRPD pattern of the crystal form H of compound 1.
FIG. 9 shows an XRPD pattern of the crystal form I of compound 1.
FIG. 10 shows an XRPD pattern of the crystal form J of compound 1.
FIG. 11 shows an XRPD pattern of the crystal form K of compound 1.
FIG. 12 shows an XRPD pattern of the amorphous form of compound 1.
FIG. 13 shows an XRPD pattern of the crystal form L of compound 1.

### DETAILED DESCRIPTION

The present disclosure is explained below in greater detail with reference to examples or experimental examples. The examples or experimental examples in the present disclosure are only illustrative of the technical solutions in the present disclosure and are not intended to limit the spirit and scope of the present disclosure.

Test conditions for the instruments used in the experiments:
The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance instrument or Bruker AVANCE NEO 500M, with dimethyl sulfoxide-D6 (DMSO-*d₆*), chloroform-D (CDCl₃), and methanol-D4 (CD₃OD) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS),
waters ACQuity UPLC-QD/SQD (manufacturer: waters; MS model: waters ACQuity Qda Detector/waters SQ Detector), and
THERMO Ultimate 3000-Q Exactive (manufacturer: THERMO; MS model: THERMO Q Exactive).

The high performance liquid chromatography (HPLC) analyses were performed using Agilent HPLC 1200DAD, Agilent HPLC 1200VWD, and Waters HPLC e2695-2489 high-pressure liquid chromatographs.

The chiral HPLC analyses were performed using an Agilent 1260 DAD high performance liquid chromatograph.

The preparative high performance liquid chromatography was performed using Waters 2545-2767, Waters 2767-SQ Detecor2, Shimadzu LC-20AP, and Gilson GX-281 preparative chromatographs. The preparative chiral chromatography was performed using a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in the thin-layer chromatography separation and purification had a layer thickness of 0.4 mm-0.5 mm.

The silica gel column chromatography generally used Yantai Huanghai silica gel with a mesh size of 200-300 as the carrier.

The mean kinase inhibition rates and IC₅₀ values were measured using a NovoStar microplate reader (BMG, Germany).

The known starting materials in the present disclosure may be synthesized by using or according to methods known in the art, or may be purchased from companies such as ABCR GmbH & Co. KG, Acros Organics, Aldrich Chemical Company, Accela ChemBio Inc., and Chembee Chemicals. In the examples, the reactions can all be performed under an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

Pressurized hydrogenation reactions were performed using a Parr 3916EKX hydrogenation instrument and a Qinglan QL-500 hydrogen generator, or an HC2-SS hydrogenation instrument. Hydrogenation reactions generally involved 3 cycles of vacuumization and hydrogen filling. Microwave reactions were performed using a CEM Discover-S 908860 microwave reactor.

In the examples, solutions were aqueous solutions unless otherwise specified.

In the examples, the reaction temperature was room temperature, i.e., 20 °C-30 °C, unless otherwise specified.

The reaction processes in the examples were monitored using thin-layer chromatography (TLC). The developing solvents used for reactions, the eluent systems of column chromatography used for compound purification, and the developing solvent systems of thin-layer chromatography included: A: a dichloromethane/methanol system, B: an *n*-hexane/ethyl acetate system, C: a petroleum ether/ethyl acetate system, and D: an ethyl acetate/methanol system. The volume ratio of the solvents was adjusted based on the polarity of the compound, or a small amount of basic or acidic reagents such as triethylamine and acetic acid could be added for adjustment.

XRPD refers to X-ray powder diffraction analysis: The analysis was performed using a BRUKER D8 X-ray diffractometer; specific acquisition information: a Cu anode (40 kV, 40 mA), ray: monochromatic Cu-Ka ray (l = 1.5418 Å). Scan mode: *θ*/2*θ*; scan range: 3-48°.

DSC refers to differential scanning calorimetry: The analysis was performed using a METTLER TOLEDO DSC 3+ differential scanning calorimeter, with a temperature ramp rate of 10 °C/min, specific temperature ranges shown in corresponding thermograms (mostly 25-300 °C or 25-350 °C), and a nitrogen purge speed of 50 mL/min.

TGA refers to thermogravimetric analysis: The analysis was performed using a METTLER TOLEDO TGA 2 thermogravimetric analyzer, with a temperature ramp rate of 10 °C/min, specific temperature ranges shown in corresponding profiles (mostly 30-400 °C), and a nitrogen purge speed of 50 mL/min.

DVS refers to dynamic vapor sorption: The analysis was performed using SMS DVS Advantage; at 25 °C, the humidity was changed as follows: 50%-95%-0%-95%-50%, in steps of 10% (5% for the last step) (specific humidity ranges are shown in corresponding patterns, and those listed here were used in most cases); the criterion was dm/dt being not greater than 0.002%.

### Example 1: Preparation of Compound Represented by Formula 1

### 1-(endo-3-((4-((4-([1,2,4]Triazolo[1,5-a]pyridin-7-yloxy)-2-fluoro-3-methylphenyl)amino)quinazolin-6-yl)oxy)-8-azabicyclo[3.2.1]octan-8-yl)prop-2-en-1-one 1

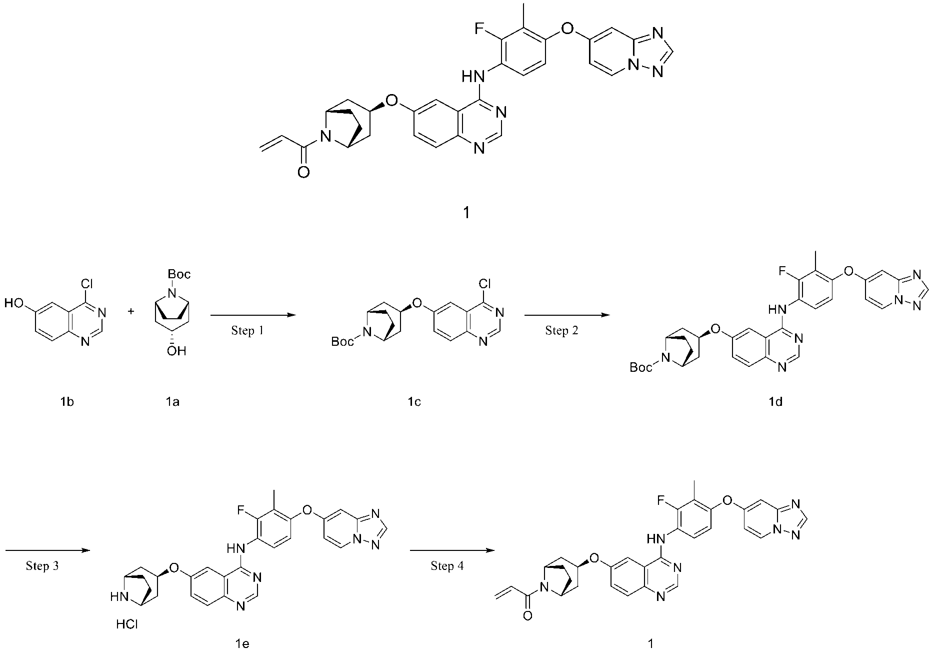

### Step 1

### tert-Butyl endo-3-((4-chloroquinazolin-6-yl)oxy)-8-azabicyclo[3.2.1]octane-8-carboxylate 1c

4-Chloroquinazolin-6-ol **1b** (20 mg, 110 µmol, prepared by the method disclosed in the literature *"*European Journal of Medicinal Chemistry, 2018, vol. 147, p. 130-149"), compound **1a** (30 mg, 133 µmol), and triphenylphosphine (58 mg, 221 µmol) were dissolved in tetrahydrofuran (5 mL), and diisopropyl azodicarboxylate (34 mg, 166 µmol) was added dropwise in an ice bath. The mixture was naturally warmed to room temperature and then allowed to react for 16 h. The reaction mixture was concentrated under reduced pressure, and then the residue was purified by silica gel column chromatography using eluent system C to give the title compound **1c** (40 mg, yield: 92.6%). MS m/z (ESI): 390.2 [M+1].

### Step 2

### tert-Butyl endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]octane-8-carboxylate 1d

Compound **1c** (1.2 g, 3 mmol) was dissolved in isopropanol (5 mL), and 4-([1,2,4]triazolo[1,5-*a*]pyridin-7-yloxy)-2-fluoro-3-methylaniline (845 mg, 3.27 mmol, prepared by the method disclosed in Example 95 on page 143 of the specification in the patent application "WO2022003575A1") was added. The mixture was allowed to react at 80 °C for 2 h. The reaction mixture was concentrated under reduced pressure, and the residue was purified by silica gel column chromatography using eluent system A to give the title compound **1d** (1.05 g, yield: 55.7%). MS m/z (ESI): 612.2 [M+1].

### Step 3

### 6-((endo-8-Azabicyclo[3.2.1]octan-3-yl)oxy)-N-(4-([1,2,4]triazolo[1,5-a]pyridin-7-yloxy)-2-fluoro-3-methylphenyl)quinazolin-4-amine hydrochloride 1e

Compound **1d** (1.05 g, 1.7 mmol) was dissolved in methanol (10 mL), and a 4 M solution of hydrogen chloride in dioxane (0.1 mL) was added. The mixture was stirred for 1 h. The reaction mixture was concentrated under reduced pressure to give a crude product of the title compound **1e** (940 mg). The crude product was directly used in the next step without purification. MS m/z (ESI): 512.2 [M+1].

### Step 4

### 1-(endo-3-((4-((4-([1,2,4]Triazolo[1,5-a]pyridin-7-yloxy)-2-fluoro-3-methylphenyl)amino)quinazolin-6-yl)oxy)-8-azabicyclo[3.2.1]octan-8-yl)prop-2-en-1-one 1

The crude product of compound **1e** (250 mg, 488.7 µmol) was dissolved in dichloromethane (5 mL), *N*,*N*-diisopropylethylamine (126 mg, 974 µmol) was added, and acryloyl chloride (50 mg, 552.4 µmol) was added in an ice bath. The mixture was stirred for 1 h. The reaction mixture was concentrated under reduced pressure, and the residue was purified by preparative high performance liquid chromatography (Waters-2545, column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compound **1** (110 mg, yield: 39.7%). According to X-ray powder diffraction analysis, the product was an amorphous form. The X-ray powder diffraction pattern of the amorphous form is shown in FIG. 12.

MS m/z (ESI): 566.2 [M+1].

¹H NMR (500 MHz, MeOD): δ 8.80-8.78 (d, 1H), 8.41 (s, 1H), 8.33 (s, 1H), 7.84-7.82 (m, 1H), 7.77 (s, 1H), 7.60-7.56 (m, 2H), 7.14-7.10 (m, 2H), 7.02-7.01 (m, 1H), 6.79-6.74 (m, 1H), 6.35 (dd, 1H), 5.81 (dd, 1H), 4.97-4.93 (m, 1H), 4.76-4.73 (m, 1H), 4.61-4.59 (m, 1H), 2.44-2.17 (m, 10H), 2.06-2.02 (m, 1H).

### Test example 1: Ba/F3 Cell Proliferation Assay

EGFR wild-type Ba/F3 cells (Cobioer, Cat. No. CBP73110) in the logarithmic growth phase were seeded into a 96-well plate at 2.5 × 10³ cells/100 µL in a growth medium, and HER2 wild-type Ba/F3 cells (Cobioer, Cat. No. CBP73110) or HER2 A775_G776insYVMA mutant Ba/F3 cells (Cobioer, Cat. No. CBP73184) in the logarithmic growth phase were seeded into a 96-well plate at 5 × 10³ cells/100 µL in a growth medium. The plates were placed in a cell incubator at 37 °C overnight. The next day, the compound diluted in a 3-fold gradient with a medium was added to the plates at 100 µL/well. All treatments were performed in triplicate. The plates were incubated in the cell incubator at 37 °C for another 72 h. Celltiter-Glo Luminescent cell viability assay was performed.

The cell proliferation rate corresponding to each well was calculated according to the following formula: proliferation% = (test compound well G3 - G0 average value)/(DMSO control well G3 average value - G0 average value) × 100. According to the proliferation rate and concentration corresponding to each gradient concentration well, a gradient curve for cell proliferation was fitted using Prism Graphpad software, and the GI50 (GI50 is defined as the compound concentration corresponding to a cell proliferation inhibition rate of 50%) of the compound was calculated.

The HER2-selective inhibitor, tucatinib (synthesized with reference to Example 11 in the patent WO2007059257A2), has the following structure:

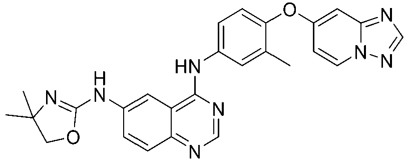

**Table 1**

| Example No. | HER2 WT/ BaF3 GI₅₀ (nM) | HER2 WT/ BaF3 Imax % | HER2 A775_G776 ins YVMA/ BaF3 GI₅₀ (nM) | HER2 A775_G776 ins YVMA/ BaF3 Imax % | EGFR WT/ BaF3 GI₅₀ (nM) | EGFR WT/ BaF3 Imax % |
|---|---|---|---|---|---|---|
| 1 | 9.3 | 100 | 10.3 | 100 | 2840 | 100 |
| Tucatinib | - | - | 309.23 | 100 | - | - |

Conclusion: The compound of the present disclosure exhibited potent proliferation inhibitory effects on Ba/F3 cells with the HER2 exon 20 YVMA insertion mutation and HER2 wild-type-dependent Ba/F3 cells, while demonstrating relatively strong selectivity relative to EGFR wild-type-dependent Ba/F3 cells; furthermore, the compound of the present disclosure showed significantly superior proliferation inhibitory activity against Ba/F3 cells with the HER2 exon 20 YVMA insertion mutation compared to tucatinib.

### Example 2: Preparation of Crystal Form A

The compound represented by formula 1 (39 g) was dissolved in methanol/ethyl acetate = 1:10 (v/v) and dried *in vacuo* to give a solid. According to X-ray powder diffraction analysis, the product was defined as crystal form A. Its XRPD pattern is shown in FIG. 1, and its characteristic peak positions are shown in Table 2.

**Table 2. XRPD data of crystal form A**

| Peak No. | 2θ value [° or degrees] | D[Å] | Relative intensity% |
|---|---|---|---|
| 1 | 8.905 | 9.9227 | 100.0 |
| 2 | 9.836 | 8.9853 | 14.0 |
| 3 | 12.375 | 7.1468 | 7.4 |
| 4 | 13.207 | 6.6984 | 16.0 |
| 5 | 17.814 | 4.9752 | 11.1 |
| 6 | 18.265 | 4.8533 | 9.3 |
| 7 | 19.863 | 4.4662 | 9.0 |
| 8 | 25.090 | 3.5464 | 7.7 |
| 9 | 26.491 | 3.3619 | 7.1 |

### Example 3: Preparation of Crystal Form B

The crystal form A of the compound represented by formula 1 (200 mg) was added to methanol (2 mL), and the mixture was stirred and filtered. The filter cake was collected, washed with methanol, and dried *in vacuo* to give a solid. According to X-ray powder diffraction analysis, the product was defined as crystal form B. Its X-ray powder diffraction data are shown in Table 3, and its X-ray powder diffraction pattern is shown in FIG. 2.

**Table 3. XRPD data of crystal form B**

| Peak No. | 2θ value [° or degrees] | D[Å] | Relative intensity% |
|---|---|---|---|
| 1 | 8.963 | 9.8588 | 100.0 |
| 2 | 9.926 | 8.9040 | 15.1 |
| 3 | 12.368 | 7.1510 | 5.6 |
| 4 | 13.219 | 6.6922 | 4.1 |
| 5 | 14.570 | 6.0745 | 0.7 |
| 6 | 15.632 | 5.6643 | 1.3 |
| 7 | 17.902 | 4.9508 | 14.1 |
| 8 | 18.404 | 4.8170 | 9.0 |
| 9 | 19.297 | 4.5959 | 1.4 |
| 10 | 19.850 | 4.4691 | 16.6 |
| 11 | 21.113 | 4.2046 | 1.3 |
| 12 | 22.049 | 4.0282 | 3.8 |
| 13 | 25.713 | 3.4619 | 1.6 |
| 14 | 26.448 | 3.3673 | 2.2 |
| 15 | 27.311 | 3.2629 | 4.0 |
| 16 | 27.774 | 3.2095 | 2.3 |
| 17 | 28.312 | 3.1497 | 4.2 |
| 18 | 29.922 | 2.9838 | 3.1 |

### Example 4: Preparation of Crystal Form C

The compound represented by formula 1 (8 mg) was added to ethanol (0.08 mL) and completely dissolved. Subsequently, the resulting solution was stirred, centrifuged, and dried *in vacuo* to give a solid. According to X-ray powder diffraction analysis, the product was defined as crystal form C. Its XRPD pattern is shown in FIG. 3, and its characteristic peak positions are shown in Table 4. Its DSC thermogram showed endothermic peaks at 86.81 °C and 132.29 °C. Its TGA profile showed a weight loss of 6.36% from 30 °C to 160 °C.

**Table 4. XRPD data of crystal form C**

| Peak No. | 2θ value [° or degrees] | D[Å] | Relative intensity% |
|---|---|---|---|
| 1 | 6.071 | 14.5455 | 9.0 |
| 2 | 8.758 | 10.0886 | 100.0 |
| 3 | 9.708 | 9.1036 | 11.9 |
| 4 | 13.299 | 6.6520 | 6.3 |
| 5 | 13.787 | 6.4177 | 17.0 |
| 6 | 15.348 | 5.7686 | 34.9 |
| 7 | 15.586 | 5.6809 | 11.4 |
| 8 | 15.848 | 5.5875 | 4.1 |
| 9 | 16.807 | 5.2708 | 6.9 |
| 10 | 17.522 | 5.0574 | 26.9 |
| 11 | 18.006 | 4.9225 | 10.3 |
| 12 | 18.228 | 4.8631 | 14.7 |
| 13 | 18.695 | 4.7425 | 8.8 |
| 14 | 20.005 | 4.4348 | 10.3 |
| 15 | 21.621 | 4.1069 | 16.4 |
| 16 | 22.070 | 4.0244 | 4.6 |
| 17 | 22.332 | 3.9777 | 5.9 |
| 18 | 23.507 | 3.7815 | 2.8 |
| 19 | 24.874 | 3.5767 | 6.8 |
| 20 | 25.346 | 3.5112 | 6.4 |
| 21 | 25.724 | 3.4604 | 7.8 |
| 22 | 26.064 | 3.4160 | 2.6 |
| 23 | 26.811 | 3.3225 | 8.6 |
| 24 | 27.115 | 3.2860 | 7.1 |
| 25 | 28.523 | 3.1268 | 1.4 |
| 26 | 28.819 | 3.0955 | 1.1 |
| 27 | 31.457 | 2.8416 | 2.6 |
| 28 | 32.055 | 2.7900 | 2.0 |
| 29 | 33.246 | 2.6927 | 1.1 |
| 30 | 33.930 | 2.6399 | 2.1 |

### Example 5: Preparation of Crystal Form C

The compound represented by formula 1 (8 mg) was added to a solvent as shown in Table 5 below and completely dissolved. Subsequently, the resulting solution was stirred, centrifuged, and dried *in vacuo* to give a solid. According to X-ray powder diffraction analysis, the product was crystal form C.

**Table 5**

| Solvent | Crystal form |
|---|---|
| 0.08 mL of ethyl acetate/ethanol = 1:1 (v/v) | C |
| 0.08 mL of tetrahydrofuran/ethanol = 2:1 (v/v) | C |
| 0.08 mL of *n*-propanol | C |

### Example 6: Preparation of Crystal Form C

The compound represented by formula 1 (8 mg) was added to *n*-propanol (0.08 mL) and completely dissolved. Then, *n*-heptane (0.24 mL) was added to induce antisolvent crystallization. The mixture was centrifuged, and the solid was collected and dried *in vacuo* to give a product. According to X-ray powder diffraction analysis, the product was crystal form C.

### Example 7: Preparation of Crystal Form D

The compound represented by formula 1 (100 mg) was added to water (5 mL). The mixture was stirred and centrifuged, and the solid was collected and dried *in vacuo* to give a product. According to X-ray powder diffraction analysis, the product was defined as crystal form D. Its XRPD pattern is shown in FIG. 4, and its characteristic peak positions are shown in Table 6. Its DSC thermogram showed an endothermic peak at 145.09 °C. Its TGA profile showed a weight loss of 3.06% from 36 °C to 180 °C. DVS testing showed that the sample had a hygroscopic weight gain of about 3.07% under normal storage conditions (i.e., 25 °C and 60% RH), a hygroscopic weight gain of about 3.23% under accelerated experimental conditions (i.e., 70% RH), and a hygroscopic weight gain of about 3.52% under extreme conditions (90% RH). Furthermore, after DVS testing, the crystal form was re-identified, and the result showed that the crystal form did not change.

**Table 6. XRPD data of crystal form D**

| Peak No. | 2θ value [° or | d[Å] | Relative |
|---|---|---|---|
| 1 | 6.989 | 12.63838 | 53.4 |
| 2 | 7.512 | 11.75868 | 14.2 |
| 3 | 10.223 | 8.64582 | 46.1 |
| 4 | 10.610 | 8.33174 | 100.0 |
| 5 | 11.281 | 7.83754 | 29.6 |
| 6 | 11.696 | 7.56019 | 3.5 |
| 7 | 11.954 | 7.39772 | 7.5 |
| 8 | 12.780 | 6.92103 | 3.1 |
| 9 | 13.310 | 6.64656 | 14.8 |
| 10 | 13.927 | 6.35378 | 64.6 |
| 11 | 14.544 | 6.08546 | 73.8 |
| 12 | 14.805 | 5.97897 | 27.7 |
| 13 | 15.962 | 5.54786 | 10.9 |
| 14 | 16.176 | 5.47491 | 12.9 |
| 15 | 16.535 | 5.35707 | 32.6 |
| 16 | 17.044 | 5.19818 | 18.1 |
| 17 | 17.476 | 5.07060 | 8.8 |
| 18 | 17.607 | 5.03302 | 2.6 |
| 19 | 18.332 | 4.83576 | 64.9 |
| 20 | 18.930 | 4.68425 | 92.4 |
| 21 | 19.170 | 4.62609 | 20.6 |
| 22 | 19.686 | 4.50602 | 16.1 |
| 23 | 20.165 | 4.39998 | 21.9 |
| 24 | 20.617 | 4.30469 | 31.0 |
| 25 | 21.313 | 4.16552 | 27.7 |
| 26 | 21.683 | 4.09535 | 8.8 |
| 27 | 22.201 | 4.00086 | 13.4 |
| 28 | 22.593 | 3.93246 | 16.6 |
| 29 | 22.820 | 3.89373 | 7.3 |
| 30 | 23.081 | 3.85031 | 3.5 |
| 31 | 23.390 | 3.80013 | 8.1 |
| 32 | 23.940 | 3.71401 | 40.1 |
| 33 | 24.358 | 3.65131 | 5.3 |
| 34 | 24.873 | 3.57688 | 24.4 |
| 35 | 25.809 | 3.44918 | 14.4 |
| 36 | 26.870 | 3.31537 | 10.7 |
| 37 | 27.526 | 3.23781 | 23.3 |
| 38 | 27.809 | 3.20553 | 14.4 |
| 39 | 28.381 | 3.14222 | 1.9 |
| 40 | 28.696 | 3.10837 | 4.4 |
| 41 | 29.613 | 3.01424 | 5.0 |
| 42 | 30.639 | 2.91555 | 3.3 |
| 43 | 30.998 | 2.88263 | 5.6 |
| 44 | 31.578 | 2.83096 | 1.4 |
| 45 | 32.288 | 2.77035 | 4.6 |
| 46 | 32.596 | 2.74488 | 1.9 |
| 47 | 33.001 | 2.71207 | 1.0 |
| 48 | 33.748 | 2.65372 | 2.8 |
| 49 | 34.687 | 2.58404 | 1.9 |
| 50 | 35.478 | 2.52822 | 1.7 |
| 51 | 36.975 | 2.42918 | 2.1 |
| 52 | 38.282 | 2.34921 | 3.2 |
| 53 | 39.250 | 2.29347 | 2.5 |

### Example 8: Preparation of Crystal Form D

The compound represented by formula 1 (8 mg) was added to a solvent as shown in Table 7 below and completely dissolved. Subsequently, the resulting solution was stirred and centrifuged, and the solid was collected and dried *in vacuo* to give a product. According to X-ray powder diffraction analysis, the product was crystal form D.

**Table 7**

| Solvent | Crystal form |
|---|---|
| 0.08 mL of dichloromethane | D |
| 0.08 mL of tetrahydrofuran | D |
| 0.08 mL of water/isopropanol = 1:10 (v/v) | D |
| 0.08 mL of water/acetone = 1:10 (v/v) | D |

### Example 9: Preparation of Crystal Form D

The compound represented by formula 1 (8 mg) was added to a solvent as shown in Table 8 below (0.8 mL). The mixture was stirred and centrifuged, and the solid was collected and dried *in vacuo* to give a product. According to X-ray powder diffraction analysis, the product was crystal form D.

**Table 8**

| Solvent | Crystal form |
|---|---|
| Isopropyl acetate | D |
| Toluene | D |
| Ethyl acetate/*n*-heptane = 1:1 (v/v) | D |

### Example 10: Preparation of Crystal Form D

The compound represented by formula 1 (8 mg) was added to a solvent as shown in Table 9 below (0.08 mL) and completely dissolved. Water (0.40 mL) was added, and then a solid precipitated. The mixture was centrifuged, and the solid was collected and dried *in vacuo* to give a product. According to X-ray powder diffraction analysis, the product was crystal form D.

**Table 9**

| Solvent | Crystal form |
|---|---|
| *n*-Propanol | D |
| Propylene glycol methyl ether | D |
| Tetrahydrofuran | D |

### Example 11: Preparation of Crystal Form D

The compound represented by formula 1 (8 mg) was added to dichloromethane (0.08 mL) and completely dissolved. A solvent as shown in Table 10 below (0.40 mL) was added, and then a solid precipitated. The mixture was centrifuged, and the solid was collected and dried *in vacuo* to give a product. According to X-ray powder diffraction analysis, the product was crystal form D.

**Table 10**

| Solvent | Crystal form |
|---|---|
| Ethyl acetate | D |
| Methyl *tert*-butyl ether | D |

### Example 12: Preparation of Crystal Form D

The compound represented by formula 1 (8 mg) was added to a solvent as shown in Table 11 below (0.08 mL) and completely dissolved. Then, volatilization was performed for crystallization to give a product. According to X-ray powder diffraction analysis, the product was crystal form D.

**Table 11**

| Solvent | Crystal form |
|---|---|
| Propylene glycol methyl ether | D |
| *N*,*N*-Dimethylformamide | D |

### Example 13: Preparation of Crystal Form D

The crystal form A of the compound represented by formula 1 (200 mg) was added to acetone (0.6 mL) and completely dissolved. The resulting solution was stirred and filtered, and the filter cake was collected, washed with acetone, and dried *in vacuo* at 45 °C for 3 h to give a product. According to X-ray powder diffraction analysis, the product was crystal form D.

### Example 14: Preparation of Crystal Form E

The compound represented by formula 1 (100 mg) was added to *n*-propanol (1 mL) and completely dissolved. Then, methyl *tert*-butyl ether (5 mL) was added to induce antisolvent crystallization. The mixture was centrifuged, and the solid was collected and dried *in vacuo* to give a product. According to X-ray powder diffraction analysis, the product was defined as crystal form E. Its XRPD pattern is shown in FIG. 5, and its characteristic peak positions are shown in Table 12. Its DSC thermogram showed endothermic peaks at 209.45 °C and 226.29 °C. Its TGA profile showed no significant weight loss. DVS testing showed that the sample had a hygroscopic weight gain of about 0.11% under normal storage conditions (i.e., 25 °C and 60% RH), a hygroscopic weight gain of about 0.15% under accelerated experimental conditions (i.e., 70% RH), and a hygroscopic weight gain of about 0.25% under extreme conditions (90% RH). Furthermore, after DVS testing, the crystal form was re-identified, and the result showed that the crystal form did not change.

**Table 12. XRPD data of crystal form E**

| Peak No. | 2θ value [° or degrees] | D[Å] | Relative intensity% |
|---|---|---|---|
| 1 | 6.751 | 13.08174 | 2.7 |
| 2 | 10.080 | 8.76817 | 48.2 |
| 3 | 10.296 | 8.58471 | 21.5 |
| 4 | 11.701 | 7.55692 | 25.3 |
| 5 | 12.776 | 6.92319 | 28.4 |
| 6 | 13.650 | 6.48195 | 8.6 |
| 7 | 14.012 | 6.31537 | 73.1 |
| 8 | 14.286 | 6.19485 | 46.2 |
| 9 | 15.958 | 5.54931 | 39.5 |
| 10 | 16.507 | 5.36607 | 20.8 |
| 11 | 17.177 | 5.15819 | 28.0 |
| 12 | 17.465 | 5.07384 | 100.0 |
| 13 | 17.696 | 5.00807 | 39.2 |
| 14 | 18.635 | 4.75775 | 81.0 |
| 15 | 18.943 | 4.68107 | 15.3 |
| 16 | 19.111 | 4.64017 | 15.8 |
| 17 | 19.583 | 4.52956 | 15.2 |
| 18 | 19.828 | 4.47409 | 25.7 |
| 19 | 20.135 | 4.40650 | 99.7 |
| 20 | 20.585 | 4.31124 | 18.0 |
| 21 | 21.144 | 4.19846 | 39.9 |
| 22 | 21.698 | 4.09251 | 24.0 |
| 23 | 22.117 | 4.01587 | 15.7 |
| 24 | 23.088 | 3.84910 | 28.6 |
| 25 | 23.310 | 3.81303 | 19.4 |
| 26 | 24.118 | 3.68700 | 7.2 |
| 27 | 24.921 | 3.57006 | 68.8 |
| 28 | 25.827 | 3.44679 | 7.3 |
| 29 | 26.493 | 3.36164 | 20.9 |
| 30 | 28.137 | 3.16888 | 2.8 |
| 31 | 28.563 | 3.12263 | 17.5 |
| 32 | 29.493 | 3.02624 | 6.3 |
| 33 | 29.813 | 2.99441 | 3.9 |
| 34 | 30.377 | 2.94017 | 3.0 |
| 35 | 31.006 | 2.88187 | 3.0 |
| 36 | 31.509 | 2.83702 | 1.5 |
| 37 | 31.991 | 2.79538 | 24.4 |
| 38 | 32.607 | 2.74392 | 5.1 |
| 39 | 33.159 | 2.69953 | 1.4 |
| 40 | 33.765 | 2.65245 | 4.2 |
| 41 | 35.290 | 2.54123 | 1.7 |
| 42 | 35.519 | 2.52538 | 2.1 |
| 43 | 36.482 | 2.46091 | 4.4 |
| 44 | 36.796 | 2.44064 | 2.9 |
| 45 | 38.254 | 2.35089 | 1.8 |
| 46 | 39.368 | 2.28692 | 2.7 |
| 47 | 42.868 | 2.10793 | 3.4 |

### Example 15: Preparation of Crystal Form E

The compound represented by formula 1 (8 mg) was added to a solvent as shown in Table 13 below (0.8 mL). The mixture was stirred and centrifuged, and the solid was collected and dried *in vacuo* to give a product. According to X-ray powder diffraction analysis, the product was crystal form E.

**Table 13**

| Solvent | Crystal form |
|---|---|
| Ethyl acetate | E |
| Acetonitrile | E |
| Methyl tert-butyl ether | E |
| *n*-Heptane | E |
| Cyclohexane | E |
| *n*-Hexane | E |
| Isopropyl ether | E |

### Example 16: Preparation of Crystal Form E

The compound represented by formula 1 (8 mg) was added to a solvent II (0.08 mL) and completely dissolved. Then, a solvent III (0.40 mL) was added, and the mixture was stirred. The solvent combinations are shown in Table 14 below. The mixture was centrifuged, and the solid was collected and dried *in vacuo* to give a product. According to X-ray powder diffraction analysis, the product was crystal form E.

**Table 14**

| Solvent II | Solvent III | Crystal form |
|---|---|---|
| *n*-Propanol | Ethyl acetate | E |
| | Acetonitrile | E |
| | Methyl *tert*-butyl ether | E |
| | Methyl isobutyl ketone | E |
| Propylene glycol methyl ether | Ethyl acetate | E |
| | Acetonitrile | E |
| | Methyl *tert*-butyl ether | E |
| | Methyl isobutyl ketone | E |
| | *n*-Heptane | E |
| Tetrahydrofuran | Ethyl acetate | E |
| | Acetonitrile | E |
| | Methyl *tert*-butyl ether | E |
| | Methyl isobutyl ketone | E |
| | *n*-Heptane | E |

### Example 17: Preparation of Crystal Form E

The compound represented by formula 1 (8 mg) was dissolved in dichloromethane (0.08 mL). Then, a solvent as shown in Table 15 below (0.24 mL) was added. The mixture was stirred and then centrifuged, and the solid was collected and dried *in vacuo* to give a product. According to X-ray powder diffraction analysis, the product was crystal form E.

**Table 15**

| Solvent | Crystal form |
|---|---|
| Methyl isobutyl ketone | E |
| Isopropyl ether | E |
| *n*-Heptane | E |

### Example 18: Preparation of Crystal Form E

The compound represented by formula 1 (8 mg) was added to a solvent as shown in Table 16 below (0.08 mL). Then, antisolvent crystallization was performed. The mixture was centrifuged, and the solid was collected and dried *in vacuo* to give a product. According to X-ray powder diffraction analysis, the product was crystal form E.

**Table 16**

| Solvent | Crystal form |
|---|---|
| Acetone | E |
| Methyl isobutyl ketone | E |

### Example 19: Preparation of Crystal Form E

The crystal form F of the compound represented by formula 1 (50 mg) was added to 2-methyltetrahydrofuran (1 mL). The mixture was stirred, and the solid was collected and dried *in vacuo* to give a product. According to X-ray powder diffraction analysis, the product was crystal form E.

### Example 20: Preparation of Crystal Form E

The crystal form A of the compound represented by formula 1 (200 mg) was dissolved in isopropanol (2 mL). Then, antisolvent crystallization was performed. The solid was collected, washed with isopropanol, and dried *in vacuo* to give a product. According to X-ray powder diffraction analysis, the product was crystal form E.

### Example 21: Preparation of Crystal Form F

The compound represented by formula 1 (8 mg) was added to water/methanol = 1:1 (v/v) (0.8 mL). The mixture was stirred and centrifuged, and the solid was collected and dried *in vacuo* to give a product. According to X-ray powder diffraction analysis, the product was defined as crystal form F. Its XRPD pattern is shown in FIG. 6, and its characteristic peak positions are shown in Table 17. Its DSC thermogram showed endothermic peaks at 140.45 °C, 154.95 °C, and 226.48 °C. Its TGA profile showed a weight loss of 4.56% from 30 °C to 180 °C.

**Table 17. XRPD data of crystal form F**

| Peak No. | 2θ value [° or degrees] | D[Å] | Relative intensity% |
|---|---|---|---|
| 1 | 10.529 | 8.3956 | 35.5 |
| 2 | 12.167 | 7.2684 | 19.7 |
| 3 | 13.759 | 6.4311 | 29.7 |
| 4 | 15.084 | 5.8689 | 17.1 |
| 5 | 15.651 | 5.6574 | 8.7 |
| 6 | 17.919 | 4.9462 | 100.0 |
| 7 | 18.778 | 4.7219 | 69.4 |
| 8 | 19.283 | 4.5993 | 12.5 |
| 9 | 19.980 | 4.4404 | 1.9 |
| 10 | 20.887 | 4.2495 | 11.8 |
| 11 | 21.255 | 4.1767 | 3.3 |
| 12 | 22.566 | 3.9371 | 14.2 |
| 13 | 23.328 | 3.8102 | 18.5 |
| 14 | 24.788 | 3.5890 | 14.6 |
| 15 | 25.666 | 3.4681 | 15.6 |
| 16 | 27.326 | 3.2611 | 6.6 |
| 17 | 30.028 | 2.9735 | 7.1 |
| 18 | 31.080 | 2.8752 | 7.2 |
| 19 | 32.165 | 2.7807 | 4.2 |
| 20 | 35.112 | 2.5537 | 3.8 |
| 21 | 37.399 | 2.4026 | 4.0 |

### Example 22: Preparation of Crystal Form F

The compound represented by formula 1 (8 mg) was dissolved in a solvent as shown in Table 18 below (0.08 mL). Then, antisolvent crystallization was performed. The mixture was centrifuged, and the solid was collected and dried *in vacuo* to give a product. According to X-ray powder diffraction analysis, the product was crystal form F.

**Table 18**

| Solvent | Crystal form |
|---|---|
| Methanol | F |
| 10% water/methanol | F |
| Acetonitrile/methanol = 1:1 (v/v) | F |

### Example 23: Preparation of Crystal Form G

The crystal form F of the compound represented by formula 1 (50 mg) was dispersed in tetrahydrofuran (1 mL). The dispersion was stirred, centrifuged, and dried *in vacuo* to collect a solid. According to X-ray powder diffraction analysis, the product was defined as crystal form G. Its X-ray powder diffraction data are shown in Table 19, and its X-ray powder diffraction pattern is shown in FIG. 7. Its DSC thermogram showed endothermic peaks at 155.21 °C and 222.61 °C. Its TGA profile showed that the compound had a weight loss of 1.75% from 40 °C to 170 °C.

**Table 19. XRPD data of crystal form G**

| Peak No. | 2θ value [° or degrees] | D[Å] | Relative intensity% |
|---|---|---|---|
| 1 | 7.090 | 12.4575 | 1.5 |
| 2 | 9.485 | 9.3169 | 1.3 |
| 3 | 10.437 | 8.4692 | 12.0 |
| 4 | 11.093 | 7.9697 | 30.2 |
| 5 | 11.362 | 7.7816 | 10.8 |
| 6 | 11.881 | 7.4428 | 11.3 |
| 7 | 12.161 | 7.2723 | 9.0 |
| 8 | 12.776 | 6.9232 | 2.8 |
| 9 | 13.989 | 6.3258 | 22.6 |
| 10 | 14.503 | 6.1025 | 5.6 |
| 11 | 14.846 | 5.9622 | 4.6 |
| 12 | 15.458 | 5.7276 | 5.2 |
| 13 | 15.946 | 5.5535 | 1.0 |
| 14 | 17.124 | 5.1740 | 38.2 |
| 15 | 17.620 | 5.0293 | 100.0 |
| 16 | 19.094 | 4.6443 | 1.7 |
| 17 | 19.601 | 4.5254 | 15.7 |
| 18 | 19.929 | 4.4516 | 18.1 |
| 19 | 20.367 | 4.3569 | 4.8 |
| 20 | 20.921 | 4.2427 | 6.2 |
| 21 | 21.374 | 4.1539 | 17.5 |
| 22 | 22.677 | 3.9180 | 26.7 |
| 23 | 23.297 | 3.8152 | 6.3 |
| 24 | 23.875 | 3.7240 | 9.8 |
| 25 | 24.540 | 3.6246 | 4.8 |
| 26 | 24.788 | 3.5889 | 3.6 |
| 27 | 25.720 | 3.4609 | 4.5 |
| 28 | 26.245 | 3.3929 | 5.8 |
| 29 | 27.155 | 3.2812 | 5.8 |
| 30 | 27.485 | 3.2426 | 2.3 |
| 31 | 28.148 | 3.1677 | 2.4 |
| 32 | 28.793 | 3.0982 | 1.9 |
| 33 | 30.016 | 2.9747 | 6.7 |
| 34 | 31.019 | 2.8807 | 5.8 |
| 35 | 32.196 | 2.7780 | 2.3 |
| 36 | 33.537 | 2.6699 | 1.3 |
| 37 | 34.580 | 2.5918 | 2.4 |
| 38 | 34.850 | 2.5723 | 4.2 |
| 39 | 35.664 | 2.5154 | 2.1 |
| 40 | 37.045 | 2.4248 | 2.1 |
| 41 | 40.083 | 2.2477 | 1.5 |

### Example 24: Preparation of Crystal Form G

The crystal form F of the compound represented by formula 1 (50 mg) was dispersed in a solvent as shown in Table 20 below (1 mL). Subsequently, the dispersion was stirred and centrifuged, and the solid was collected and dried *in vacuo* to give a solid. According to X-ray powder diffraction analysis, the product was crystal form G.

**Table 20**

| Solvent | Crystal form |
|---|---|
| 2-Butanone | G |
| Methyl isobutyl ketone | G |

### Example 25: Preparation of Crystal Form H

The compound represented by formula 1 (100 mg) was dissolved in 2-butanone (1 mL). The resulting solution was stirred and centrifuged, and the solid was collected and dried *in vacuo* to give a product. According to X-ray powder diffraction analysis, the product was defined as crystal form H. Its XRPD pattern is shown in FIG. 8, and its characteristic peak positions are shown in Table 21. Its DSC thermogram showed endothermic peaks at 145.94 °C and 226.47 °C. Its TGA profile showed a weight loss of 2.96% from 37 °C to 180 °C. DVS testing showed that the sample had a hygroscopic weight gain of about 0.09% under normal storage conditions (i.e., 25 °C and 60% RH), a hygroscopic weight gain of about 0.12% under accelerated experimental conditions (i.e., 70% RH), and a hygroscopic weight gain of about 0.22% under extreme conditions (90% RH).

**Table 21. XRPD data of crystal form H**

| Peak No. | 2θ value [° or degrees] | D[Å] | Relative intensity% |
|---|---|---|---|
| 1 | 10.963 | 8.0640 | 41.2 |
| 2 | 11.405 | 7.7523 | 100.0 |
| 3 | 12.273 | 7.2059 | 28.6 |
| 4 | 13.484 | 6.5612 | 4.0 |
| 5 | 14.295 | 6.1908 | 2.0 |
| 6 | 14.569 | 6.0749 | 31.2 |
| 7 | 15.554 | 5.6925 | 13.1 |
| 8 | 17.174 | 5.1591 | 94.0 |
| 9 | 17.434 | 5.0825 | 6.3 |
| 10 | 17.759 | 4.9905 | 2.2 |
| 11 | 18.160 | 4.8811 | 2.0 |
| 12 | 19.341 | 4.5855 | 7.1 |
| 13 | 20.183 | 4.3962 | 8.9 |
| 14 | 20.715 | 4.2844 | 42.1 |
| 15 | 21.265 | 4.1749 | 28.7 |
| 16 | 21.328 | 4.1627 | 11.6 |
| 17 | 21.946 | 4.0469 | 11.1 |
| 18 | 22.626 | 3.9267 | 27.9 |
| 19 | 22.834 | 3.8914 | 21.3 |
| 20 | 23.287 | 3.8167 | 15.5 |
| 21 | 24.261 | 3.6657 | 5.1 |
| 22 | 24.600 | 3.6160 | 35.2 |
| 23 | 24.820 | 3.5844 | 6.2 |
| 24 | 25.969 | 3.4283 | 3.7 |
| 25 | 26.416 | 3.3713 | 5.2 |
| 26 | 26.638 | 3.3437 | 9.8 |
| 27 | 27.581 | 3.2315 | 16.3 |
| 28 | 28.109 | 3.1720 | 4.7 |
| 29 | 29.292 | 3.0465 | 4.1 |
| 30 | 29.433 | 3.0322 | 6.8 |
| 31 | 29.941 | 2.9820 | 2.0 |
| 32 | 30.944 | 2.8875 | 16.9 |
| 33 | 31.315 | 2.8541 | 2.1 |
| 34 | 31.878 | 2.8050 | 2.8 |
| 35 | 32.106 | 2.7856 | 10.1 |
| 36 | 33.405 | 2.6803 | 3.3 |
| 37 | 35.738 | 2.5104 | 2.3 |
| 38 | 37.185 | 2.4160 | 3.4 |

### Example 26: Preparation of Crystal Form H

The compound represented by formula 1 (8 mg) was dissolved in a solvent as shown in Table 22 below (0.08 mL). Then, antisolvent crystallization was performed. The mixture was centrifuged, and the solid was collected and dried *in vacuo* to give a product. According to X-ray powder diffraction analysis, the product was crystal form H.

**Table 22**

| Solvent | Crystal form |
|---|---|
| Isopropanol | H |
| Water/ethanol = 0.7:9.3 (v/v) | H |

### Example 27: Preparation of Crystal Form H

The compound represented by formula 1 (8 mg) was dissolved in dichloromethane (0.08 mL). Then, acetonitrile (0.40 mL) was added to induce antisolvent crystallization. The mixture was centrifuged, and the solid was collected and dried *in vacuo* to give a product. According to X-ray powder diffraction analysis, the product was crystal form H.

### Example 28: Preparation of Crystal Form I

The compound represented by formula 1 (8 mg) was added to water/methanol = 8:2 (v/v) (0.8 mL). The mixture was stirred to give a product. According to X-ray powder diffraction analysis, the wet sample of the product was defined as crystal form I. Its XRPD pattern is shown in FIG. 9, and its characteristic peak positions are shown in Table 23.

**Table 23. XRPD data of crystal form I**

| Peak No. | 2θ value [° or degrees] | d[Å] | Relative intensity% |
|---|---|---|---|
| 1 | 8.451 | 10.45476 | 100.0 |
| 2 | 9.371 | 9.43036 | 38.7 |
| 3 | 10.315 | 8.56921 | 8.3 |
| 4 | 12.950 | 6.83099 | 38.1 |
| 5 | 13.556 | 6.52682 | 18.0 |
| 6 | 14.347 | 6.16850 | 3.7 |
| 7 | 15.468 | 5.72379 | 22.1 |
| 8 | 17.504 | 5.06238 | 18.3 |
| 9 | 17.911 | 4.94842 | 16.8 |
| 10 | 19.681 | 4.50707 | 11.4 |
| 11 | 21.131 | 4.20113 | 11.1 |
| 12 | 21.787 | 4.07600 | 14.8 |
| 13 | 22.662 | 3.92052 | 2.6 |
| 14 | 24.693 | 3.60257 | 31.8 |
| 15 | 26.353 | 3.37920 | 23.4 |
| 16 | 27.632 | 3.22559 | 3.7 |

### Example 29: Preparation of Crystal Form J

The compound represented by formula 1 (30 mg) was added to 80% water/methanol = 8:2 (v/v) (3 mL). The mixture was stirred and centrifuged, and the solid was collected and dried *in vacuo* to give a product. According to X-ray powder diffraction analysis, the product was defined as crystal form J. Its XRPD pattern is shown in FIG. 10, and its characteristic peak positions are shown in Table 24. Its DSC thermogram showed endothermic peaks at 128.14 °C and 226.15 °C, and an exothermic peak at 207.16 °C. Its TGA profile showed a weight loss of 5.08% from 31 °C to 160 °C. DVS testing showed that the sample had a hygroscopic weight gain of about 2.44% under normal storage conditions (i.e., 25 °C and 60% RH), a hygroscopic weight gain of about 2.64% under accelerated experimental conditions (i.e., 70% RH), and a hygroscopic weight gain of about 8.10% under extreme conditions (90% RH).

**Table 24. XRPD data of crystal form J**

| Peak No. | 2θ value [° or degrees] | d[Å] | I Relative intensity% |
|---|---|---|---|
| 1 | 6.126 | 14.41678 | 16.5 |
| 2 | 6.746 | 13.09328 | 18.0 |
| 3 | 9.020 | 9.79573 | 100.0 |
| 4 | 10.008 | 8.83090 | 41.3 |
| 5 | 10.247 | 8.62552 | 37.0 |
| 6 | 12.315 | 7.18128 | 7.0 |
| 7 | 13.149 | 6.72781 | 37.1 |
| 8 | 13.617 | 6.49754 | 26.6 |
| 9 | 14.205 | 6.23012 | 18.7 |
| 10 | 15.473 | 5.72230 | 16.6 |
| 11 | 15.973 | 5.54399 | 25.9 |
| 12 | 17.472 | 5.07158 | 26.1 |
| 13 | 18.317 | 4.83955 | 26.1 |
| 14 | 18.631 | 4.75882 | 57.8 |
| 15 | 20.232 | 4.38573 | 36.9 |
| 16 | 22.443 | 3.95825 | 5.3 |
| 17 | 23.333 | 3.80935 | 12.0 |
| 18 | 24.977 | 3.56212 | 37.7 |
| 19 | 26.623 | 3.34554 | 46.6 |

### Example 30: Preparation of Crystal Form K

The compound represented by formula 1 (8 mg) was dissolved in acetonitrile (0.8 mL). Then, volatilization was performed for crystallization to give a product. According to X-ray powder diffraction analysis, the product was defined as crystal form K. Its XRPD pattern is shown in FIG. 11, and its characteristic peak positions are shown in Table 25.

**Table 25. XRPD data of crystal form K**

| Peak No. | 2θ value [° or degrees] | d[Å] | Relative intensity% |
|---|---|---|---|
| 1 | 5.814 | 15.18970 | 100.0 |
| 2 | 11.642 | 7.59527 | 13.7 |
| 3 | 13.010 | 6.79945 | 16.4 |
| 4 | 15.159 | 5.84005 | 15.2 |
| 5 | 17.382 | 5.09776 | 8.7 |
| 6 | 20.326 | 4.36551 | 24.9 |
| 7 | 21.274 | 4.17315 | 4.4 |
| 8 | 23.543 | 3.77579 | 27.4 |
| 9 | 25.257 | 3.52335 | 2.6 |
| 10 | 25.976 | 3.42744 | 3.0 |
| 11 | 28.101 | 3.17288 | 5.6 |
| 12 | 30.296 | 2.94777 | 5.8 |

### Example 31: Preparation of Crystal Form L

The compound represented by formula 1 (0.5 g) was dissolved in acetone. The resulting solution was stirred at room temperature for 16 h to give a solid. According to X-ray powder diffraction analysis, the product was defined as crystal form L. Its XRPD pattern is shown in FIG. 13, and its characteristic peak positions are shown in Table 26.

**Table 26**

| Peak No. | 2θ value [° or degrees] | D[Å] | Relative intensity% |
|---|---|---|---|
| 1 | 5.350 | 16.50523 | 19.4 |
| 2 | 7.471 | 11.82280 | 41.0 |
| 3 | 8.324 | 10.61345 | 6.0 |
| 4 | 10.514 | 8.40751 | 27.4 |
| 5 | 12.171 | 7.26595 | 9.3 |
| 6 | 12.908 | 6.85260 | 17.1 |
| 7 | 13.476 | 6.56532 | 51.2 |
| 8 | 13.876 | 6.37670 | 31.0 |
| 9 | 15.802 | 5.60370 | 19.0 |
| 10 | 16.708 | 5.30198 | 91.9 |
| 11 | 17.950 | 4.93768 | 29.4 |
| 12 | 18.749 | 4.72895 | 100.0 |
| 13 | 20.247 | 4.38237 | 4.2 |
| 14 | 21.243 | 4.17906 | 37.0 |
| 15 | 21.932 | 4.04932 | 5.9 |
| 16 | 23.202 | 3.83052 | 18.2 |
| 17 | 24.826 | 3.58355 | 7.6 |
| 18 | 26.097 | 3.41173 | 17.5 |
| 19 | 29.531 | 3.02237 | 4.3 |
| 20 | 29.881 | 2.98778 | 7.3 |

### Example 32: Influencing Factor Stability Study

Samples of the crystal forms D, E, and H were spread in open containers and left to stand under light exposure (4500 Lux), high temperature (40 °C and 60 °C), and high humidity (RH 75% and RH 92.5%) conditions for 1 month to investigate their stability.

**Table 27. Influencing factor stability of crystal form D**

| **Condition** | **Time (days)** | **Crystal form D** | | |
|---|---|---|---|---|
| | | **Color and appearance** | **Purity (%)** | **Crystal form** |
| Initial | 0 | White powder | 99.6 | Crystal form D |
| 40 °C | 7 days | White powder | 99.5 | No change |
| | 13 days | White powder | 99.4 | No change |
| | 1 month | White powder | 99.0 | No change |
| 60 °C | 7 days | White powder | 99.5 | No change |
| | 13 days | White powder | 99.4 | No change |
| | 1 month | White powder | 99.2 | No change |
| 4500 Lux | 7 days | White powder | 99.4 | No change |
| | 13 days | White powder | 99.3 | No change |
| | 1 month | White powder | 99.2 | No change |
| 75% RH | 13 days | White powder | 99.6 | No change |
| | 1 month | White powder | 99.6 | No change |
| 92.5% RH | 13 days | White powder | 99.6 | No change |
| | 1 month | White powder | 99.6 | No change |

| | | | | |
|---|---|---|---|---|
| Conclusion: Standing under light exposure (4500 Lux), high temperature (40 °C and 60 °C), and high humidity (RH 75% and RH 92.5%) conditions for 30 days, the crystal form D exhibited good physical and chemical stability. | | | | |

**Table 28. Influencing factor stability of crystal form E**

| **Condition** | **Time (days)** | **Crystal form E** | | |
|---|---|---|---|---|
| | | **Color and appearance** | **Purity (%)** | **Crystal form** |
| Initial | 0 | White powder | 99.7 | Crystal form E |
| 40 °C | 7 days | White powder | 99.6 | No change |
| | 14 days | White powder | 99.6 | No change |
| | 1 month | White powder | 99.3 | No change |
| 60 °C | 7 days | White powder | 99.7 | No change |
| | 14 days | White powder | 99.7 | No change |
| | 1 month | White powder | 99.5 | No change |
| 4500 Lux | 7 days | White powder | 99.6 | No change |
| | 14 days | White powder | 99.5 | No change |
| | 1 month | White powder | 99.4 | No change |
| 75% RH | 7 days | White powder | 99.7 | No change |
| | 14 days | White powder | 99.7 | No change |
| | 1 month | White powder | 99.7 | No change |
| 92.5% RH | 7 days | White powder | 99.7 | No change |
| | 14 days | White powder | 99.7 | No change |
| | 1 month | White powder | 99.7 | No change |

| | | | | |
|---|---|---|---|---|
| Conclusion: Standing under light exposure (4500 Lux), high temperature (40 °C and 60 °C), and high humidity (RH 75% and RH 92.5%) conditions for 30 days, the crystal form E exhibited good physical and chemical stability. | | | | |

**Table 29. Influencing factor stability of crystal form H**

| **Condition** | **Time (days)** | **Crystal form H** | | |
|---|---|---|---|---|
| | | **Color and appearance** | **Purity (%)** | **Crystal form** |
| Initial | 0 | White powder | 99.8 | Crystal form H |
| 40 °C | 7 days | White powder | 99.6 | No change |
| | 14 days | White powder | 99.6 | No change |
| | 1 month | White powder | 99.4 | No change |
| 60 °C | 7 days | White powder | 99.7 | No change |
| | 14 days | White powder | 99.6 | No change |
| | 1 month | White powder | 99.4 | No change |
| 4500 Lux | 7 days | White powder | 99.7 | No change |
| | 14 days | White powder | 99.7 | No change |
| | 1 month | White powder | 99.7 | No change |
| 75% RH | 7 days | White powder | 99.7 | No change |
| | 14 days | White powder | 99.7 | No change |
| | 1 month | White powder | 99.7 | No change |
| 92.5% RH | 7 days | White powder | 99.7 | No change |
| | 14 days | White powder | 99.7 | No change |
| | 1 month | White powder | 99.7 | No change |

| | | | | |
|---|---|---|---|---|
| Conclusion: Standing under light exposure (4500 Lux), high temperature (40 °C and 60 °C), and high humidity (RH 75% and RH 92.5%) conditions for 30 days, the crystal form H exhibited good physical and chemical stability. | | | | |

### Example 33: Long-Term/Accelerated Stability

Samples of the crystal forms D, E, and H were left to stand under 25 °C/60% RH and 40 °C/75% RH conditions to investigate their stability:

**Table 30. Long-term/accelerated stability of crystal form D**

| **Condition** | **Time (days)** | **Crystal form D** | | |
|---|---|---|---|---|
| | | **Color and appearance** | **Purity (%)** | **Crystal form** |
| Initial | 0 | White powder | 99.6 | Crystal form D |
| 25 °C/60% RH | 14 days | White powder | 99.6 | No change |
| | 1 month | White powder | 99.6 | No change |
| | 2 months | White powder | 99.5 | No change |
| | 3 months | White powder | 99.5 | No change |
| | 6 months | White powder | 99.5 | No change |
| 40 °C/75% RH | 14 days | White powder | 99.5 | No change |
| | 1 month | White powder | 99.6 | No change |
| | 2 months | White powder | 99.5 | No change |
| | 3 months | White powder | 99.5 | No change |
| | 6 months | White powder | 99.5 | No change |

| | | | | |
|---|---|---|---|---|
| Conclusion: Under long-term/accelerated conditions, the crystal form D exhibited good physical and chemical stability. | | | | |

**Table 31. Long-term/accelerated stability of crystal form E**

| **Condition** | **Time (days)** | **Crystal form E** | | |
|---|---|---|---|---|
| | | **Color and appearance** | **Purity (%)** | **Crystal form** |
| Initial | 0 | White powder | 99.7 | Crystal form E |
| 25 °C/60% RH | 14 days | White powder | 99.7 | No change |
| | 1 month | White powder | 99.7 | No change |
| | 2 months | White powder | 99.7 | No change |
| | 3 months | White powder | 99.7 | No change |
| | 6 months | White powder | 99.6 | No change |
| 40 °C/75% RH | 14 days | White powder | 99.7 | No change |
| | 1 month | White powder | 99.7 | No change |
| | 2 months | White powder | 99.7 | No change |
| | 3 months | White powder | 99.7 | No change |
| | 6 months | White powder | 99.7 | No change |

| | | | | |
|---|---|---|---|---|
| Conclusion: Under long-term/accelerated conditions, the crystal form E exhibited good physical and chemical stability. | | | | |

**Table 32. Long-term/accelerated stability of crystal form H**

| **Condition** | **Time (days)** | **Crystal form H** | | |
|---|---|---|---|---|
| | | **Color and appearance** | **Purity (%)** | **Crystal form** |
| Initial | 0 | White powder | 99.8 | Crystal form H |
| 25 °C/60% RH | 14 days | White powder | 99.7 | No change |
| | 1 month | White powder | 99.7 | No change |
| | 2 months | White powder | 99.7 | No change |
| | 3 months | White powder | 99.7 | No change |
| | 6 months | White powder | 99.8 | No change |
| 40 °C/75% RH | 14 days | White powder | 99.7 | No change |
| | 1 month | White powder | 99.7 | No change |
| | 2 months | White powder | 99.7 | No change |
| | 3 months | White powder | 99.7 | No change |
| | 6 months | White powder | 99.8 | No change |

| | | | | |
|---|---|---|---|---|
| Conclusion: Under long-term/accelerated conditions, the crystal form H exhibited good physical and chemical stability. | | | | |

## Claims

1. A crystal form E of the compound represented by formula 1, wherein an X-ray powder diffraction pattern expressed in terms of 2*θ* angles, which are diffraction angles, of the crystal form has characteristic peaks at 10.080, 14.012, 17.465, 18.635, 20.135, and 24.921, preferably at 10.080, 11.701, 12.776, 14.012, 15.958, 17.465, 17.696, 20.135, 21.144, 23.088, and 24.921, and more preferably at 10.080, 10.296, 11.701, 12.776, 14.012, 14.286, 15.958, 16.507, 17.177, 17.465, 17.696, 18.635, 19.111, 19.583, 19.828, 20.135, 21.144, 21.698, 23.088, 23.310, 24.921, 26.493, 28.563, and 31.991,

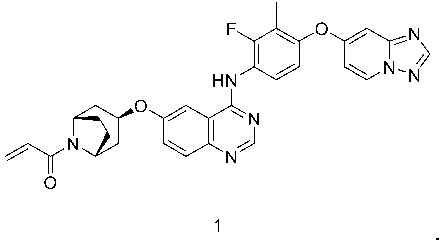

2. The crystal form E according to claim 1, wherein the X-ray powder diffraction pattern expressed in terms of 2*θ* angles, which are diffraction angles, of the crystal form is shown in FIG. 5.

3. A method for preparing the crystal form E according to claim 1 or 2, comprising:
method I: adding the compound represented by formula 1 to a solvent I and stirring, wherein the solvent I is one or more solvents selected from the group consisting of ethyl acetate, acetonitrile, *n*-heptane, methyl *tert*-butyl ketone, isopropanol, 1,4-dioxane, 2-methyltetrahydrofuran, acetone, methyl *tert-*butyl ether, cyclohexane, *n*-hexane, and isopropyl ether;
method II: dissolving the compound represented by formula 1 in a solvent II, adding a solvent III, and stirring, wherein when the solvent II is selected from the group consisting of *n*-propanol, the solvent III added is a solvent selected from the group consisting of ethyl acetate, acetonitrile, methyl *tert*-butyl ether, and methyl isobutyl ketone; when the II is selected from the group consisting of propylene glycol methyl ether and tetrahydrofuran, the solvent III added is a solvent selected from the group consisting of ethyl acetate, acetonitrile, methyl *tert*-butyl ether, methyl isobutyl ketone, and *n*-heptane; when the II is selected from the group consisting of dichloromethane, the solvent III added is a solvent selected from the group consisting of isopropyl ether, methyl isobutyl ketone, and *n*-heptane.

4. A crystal form D of the compound represented by formula 1, wherein an X-ray powder diffraction pattern expressed in terms of 2*θ* angles, which are diffraction angles, of the crystal form has characteristic peaks at 6.989, 10.610, 13.927, 14.544, 18.332, 18.930, and 23.940, preferably at 6.989, 7.512, 10.223, 10.610, 11.281, 13.927, 14.544, 14.805, 16.535, 18.332, 18.930, and 23.940, and more preferably at 6.989, 7.512, 10.223, 10.610, 11.281, 13.310, 13.927, 14.544, 14.805, 16.535, 17.044, 18.332, 18.930, 20.617, 21.313, and 23.940.

5. The crystal form D according to claim 1, wherein the X-ray powder diffraction pattern expressed in terms of 2*θ* angles, which are diffraction angles, of the crystal form is shown in FIG. 4.

6. A method for preparing the crystal form D according to claim 4 or 5, comprising:
method I: adding the compound represented by formula 1 to a solvent V and stirring, wherein the solvent V is one or more solvents selected from the group consisting of water, isopropyl acetate, toluene, and ethyl acetate/*n*-heptane = 1:1 (v/v);
method II: dissolving the compound represented by formula 1 in a solvent VI and stirring, wherein the solvent VI is one or more solvents selected from the group consisting of dichloromethane, tetrahydrofuran, water/isopropanol = 1:9 (v/v), and water/acetone = 1:9 (v/v);
method III: dissolving the compound represented by formula 1 in a solvent VII, adding a solvent VIII, and stirring, wherein when the VII is a solvent selected from the group consisting of *n-*propanol, propylene glycol methyl ether, and tetrahydrofuran, the solvent VIII added is water; when the VII is selected from the group consisting of dichloromethane, the solvent VIII added is a solvent selected from the group consisting of ethyl acetate and methyl *tert*-butyl ether;
method IV: dissolving the compound represented by formula 1 in a solvent IX and volatilizing for crystallization, wherein the solvent IX is one or more solvents selected from the group consisting of propylene glycol methyl ether and *N*,*N*-dimethylformamide.

7. A crystal form H of the compound represented by formula 1, wherein an X-ray powder diffraction pattern expressed in terms of 2*θ* angles, which are diffraction angles, of the crystal form has characteristic peaks at 10.963, 11.405, 17.174, 20.715, 21.265, and 24.600, preferably at 10.963, 11.405, 12.273, 14.569, 17.174, 20.715, 21.265, 21.328, 22.626, 22.834, and 24.600, and more preferably at 10.963, 11.405, 12.273, 14.569, 17.174, 20.715, 21.265, 21.328, 22.626, 22.834, 24.600, 27.581, and 30.944.

8. The crystal form H according to claim 1, wherein the X-ray powder diffraction pattern expressed in terms of 2*θ* angles, which are diffraction angles, of the crystal form is shown in FIG. 8.

9. A method for preparing the crystal form H according to claim 7 or 8, comprising:
method I: dissolving the compound represented by formula 1 in water/ethanol = 0.7:9.3 (v/v), isopropanol, or 2-butanone, and stirring;
method II: dissolving the compound represented by formula 1 in dichloromethane, adding acetonitrile, and stirring.

10. The crystal form according to any one of claims 1-2, 4-5, and 7-8, wherein the 2*θ* angles have a margin of error of ±0.20.

11. A pharmaceutical composition, comprising the crystal form according to any one of claims 1-2, 4-5, and 7-8, and optionally a pharmaceutically acceptable excipient.

12. A method for preparing a pharmaceutical composition, comprising a step of mixing the crystal form according to any one of claims 1-2, 4-5, and 7-8 with a pharmaceutically acceptable excipient.

13. Use of the crystal form according to any one of claims 1-2, 4-5, and 7-8, or the pharmaceutical composition according to claim 11 in the manufacture of a medicament for treating and/or preventing a cancer or tumor.
